# EUROPEAN PATENT SPECIFICATION

(11) **EP 2 398 843 B1**
(45) Date of publication and mention of the grant of the patent: **17.07.2013**
(21) Application number: 10706833.0
(22) Date of filing: 19.02.2010
(51) Int. Cl.: C08J 3/00

(54) **HYDROPHILIC APERTURED FORMED FILM**
HYDROPHILE MIT GEFORMTE FOLIE MIT ÖFFNUNGEN
FILM FAÇONNÉ AJOURÉ HYDROPHILE

(30) Priority: 20.02.2009 US 208109 P; 27.02.2009 US 208734 P
(43) Date of publication of application: 28.12.2011
(73) Proprietor: Tredegar Film Products Corporation, Richmond, VA 23225 (US)
(72) Inventor: MASCHINO, Andrew, D., Paris, IL 61944 (US); GROSS, Alvin, W., Terre Haute, IN 47803 (US)
(74) Representative: Nederlandsch Octrooibureau
(86) International application number: PCT/US2010/024644
(87) International publication number: WO 2010/096601

(56) References cited:
- WO-A1-00/09597
- WO-A1-2008/045579
- JP-A- 2002 331 024
- US-A1- 2007 191 511
- US-A1- 2009 041 820

## Description

### Background

The disclosure relates to disposable absorbent products and, more particularly, to an apertured vacuum formed or hydroformed three-dimensional film having properties that give the film a hydrophilic affinity to liquids.

Apertured films are plastic films that are processed to create apertures or holes in the film. Apertured films fall into two general categories; three-dimensional films and flat films. While all films have three-dimensions in that they have a nominal thickness, a "three-dimensional film" (also known and referred to herein as "formed films") in the context of the present disclosure is one having surface structures or protuberances that extend from the film surface and terminate in an aperture. These protuberances provide a greater thickness to the film beyond the nominal thickness of the film. Such films are characterized by a thickness or loft defined as the distance between the base plane of the film and a secondary plane defined by the apertures at the terminal end of the protuberance. The secondary plane is spaced from and generally parallel to the base plane of the film. In multi-planar embodiments, additional protuberances can originate from the surfaces in the secondary plane, forming a third plane in the film. In general, the protuberances in have a memory in that they will resume their shape after being deformed by pressure or tension, so long as the deformation limit of the film has not been compromised. By contrast, flat apertured films are simply films with holes and lack the surface structures with three-dimensional memory that characterize "three-dimensional films".

There are several processes in the art for making apertured films. Flat films can be made by any process in which holes are added to the film. In general, these are mechanical processes using pins or other embossments to create holes in a precursor film. For three-dimensional films, the two most common processes are vacuum forming and hydroforming. The vacuum forming process, exemplified by references such as US 3939135 and US 4324246, uses a vacuum to create a negative pressure on one side of a film and a corresponding positive pressure on the opposite side of the film. The pressure differential causes film to be drawn into a hole in the forming screen to create the apertures. In a hydroforming process, as exemplified by the reference US 4609518, high pressure water jets are used to generate streams of liquid that impinge upon the film, thus pushing the film into a hole in the forming screen to create the apertures.

In either the hydroforming or vacuum forming process, the film is supported on a perforated structure known in the art, and referenced herein, as a forming screen. In a direct cast process, a molten polymer is extruded through a die onto the forming screen and then subjected to the vacuum. Such a process is exemplified by US 4456570, incorporated herein by reference. In a reheat process, a precursor film is heated to a point above the softening point but below the melting point of the film, and then subjected to the vacuum. The precursor film can be a cast or blown film. The reheat process is exemplified by US 4151240, incorporated herein by reference.

Other types of apertured films may include, but are not limited to hydroformed films, exemplified by U.S. Pat. Nos. 4609518; 4629643; 4695422; 4772444; 4778644; 4839216; and 4637819, all of which are incorporated herein by reference. The hydroforming process is similar to the reheat process, except that high pressure water jets are used to force the film into the openings in the screen and thus form the apertured protuberances. It is often the case that a vacuum is also applied to the film to assist in the removal and recycling of the water, but otherwise plays no role in the formation of the protuberances.

Over the years, it was discovered that the functionality of a three-dimensional film as a topsheet for absorbent devices was enhanced if surfactants were added. A "surfactant" is a chemical species that act as wetting agents to lower the surface tension of a liquid and allow for increased spreadability of the liquid. Common uses in polymer film products are anti-fog and antistatic applications. Because the films were made from naturally hydrophobic polymers such as thermoplastic polyolefins, the addition of a surfactant to the films improved the wettability of the films which in turn caused fluids to spread on the films. In hygiene applications, this was beneficial because it made the films feel dryer to the user and improved fluid flow into the apertures. Well known anti-fogging agents are alkylphenol ethoxylates, complex polyol monoesters, polyoxyethylene esters of oleic acid, polyoxyethylene sorbitan esters of oleic acid, and sorbitan esters of fatty acids.

At first, the common practice was to apply a surfactant topically to the film, but current best practice is to incorporate the surfactant into the resin mixture to produce films having a hydrophilic affinity for liquids. Such a method is exemplified by the aforementioned US 4456570. Modem surfactants useful in formed films for hygiene applications can be found in US 5520875 and comprise non-ionic surfactants such as: alcohol ethoxylates, alkylphenol ethoxylates, carboxylic acid esters, glycerol esters, polyoxyethylene esters of fatty acids, polyoxyethylene esters of aliphatic carboxylic acids related to abietic acid, anhydrosorbitol esters, ethoxylated anhydrosorbitol esters, ethoxylated natural fats, oils, and waxes, glycol esters of fatty acids, carboxylic amides, diethanolamine condensates, monoalkanolamine condensates, polyoxyethylene fatty acid amides, polyalkyleneoxide block copolymers. Such surfactants will migrate to the surface of the film, or "bloom" after the film is formed to impart hydrophilic affinity to the surface of the film. US 6353149 discloses that surfactants selected from the group consisting of ethoxylated fatty alcohols, ethoxylated alkyl phenols, ethoxylated mono fatty acid esters, ethoxylated di fatty esters, and mixtures thereof show an accelerated migration.

In hospital operating rooms where alcohol and other flammable solvents are prevalent, it is paramount that any static sparks are avoided which could ignite those solvents. An antistatic agent is a compound used for treatment of materials or their surfaces in order to reduce or eliminate buildup of static electricity. Its role is to make the surface or the material itself slightly conductive by becoming conductive itself by absorbing moisture from the air. Often the molecules of an antistatic agent have both hydrophilic and hydrophobic areas; the hydrophobic side interacts with the surface of the material, while the hydrophilic side interacts with the air moisture and binds the water molecules to form a thin sheet of water (as with the anti-fog surfactants). This thin sheet of water then conducts the static charge to a ground such that it can not grow in order to discharge a static spark. Many antistatic agents are based on long-chain aliphatic amines (optionally ethoxylated), amides, esters of phosphoric acid, polyethylene glycol esters, or polyols.

Fatty alcohols and other fatty elements in the prior art are 'ethoxylated' in order to be used as surfactants. Narrow-range ethoxylates ("NRE") are known nonionic surfactants which are produced by the addition of ehtylene oxide onto fatty alcohols in the presence of suitable catalysts (layer compounds which have been calcined or hydrophobized with fatty acids). These surfactant chemistries are well known in the art.

Unaltered fatty alcohols can be found in absorptive device prior art but they are components in mixtures used for lotions and coatings to enhance dryness or promote skin health. Examplary arts include US 6426444; US 6503526; and US 2008/0287896, all of which are incoprporated herein by reference.

While multiple components derived from the fatty substances of plants or animals are common in the formed film topsheet prior art as surfactants and lotion, fatty alcohols were not previously used as migrating surfactants in extruded resin blends until the art of this invention.
American patent US2009/041820 discloses an extruded film with a thermoplastic olefm polymer and a fatty alcohol, suitable for a wide range of applications including woven and non-woven fabrics. The functional polymer composition includes at least 50 wt% of one or more polymer components, from 0.1 to 50 wt% of one or more fluids and from 1 to 50% of one or more active substrates.
International Patent publication WO2008/045579 discloses a method for coating a Chinese medicine by encapsulation, agglomeration and/or absorption.
American patent US2007/191511 discloses a pigmented thermoplastic material.
International patent publication WO 00/09597 discloses a puncture-resistant polyolefin membrane, made of semi-crystalline thermoplastic polymers.
Japanese patent publication JP-2002-331024 discloses a laminating material and an adhesive plaster, made of a thermoplastic resin, wherein the layer contacting the skin is a olefin thermoplastic elastomer.

### Summary Of The Disclosure

In one embodiment, the disclosure provides a three-dimensional apertured film as claimed in claim 1 comprising at least a hydrophobic thermoplastic polymer and a fatty alcohol in an amount sufficient to render the film hydrophilic.

In another embodiment, the disclosure provides an absorbent article as described in claim 6 comprising a topsheet and an absorbent core, wherein the topsheet comprises a three-dimensional apertured film comprising at least a hydrophobic thermoplastic polymer and a fatty alcohol in an amount sufficient to render the film hydrophilic.

In another embodiment, the disclosure provides an absorbent article as described in claim 7 comprising a topsheet, an absorbent core and a transfer layer located between the topsheet and the core, wherein at least one of the topsheet and the transfer layer comprises a three-dimensional apertured film comprising at least a hydrophobic thermoplastic polymer and a fatty alcohol in an amount sufficient to render the film hydrophilic.

These and other aspects of the disclosure will become apparent upon a further reading of the specification with reference to the drawings and the appended claims.

### Detailed Description

Fatty alcohols are aliphatic alcohols derived from natural fats and oils, primarily those originating in plants. Fatty alcohols usually have an even number of carbon atoms. Production from fatty acids yields normal-chain alcohols, or stated differently, the alcohol group (-OH) attaches to the terminal carbon. Other processing can yield isoalcohols where the alcohol group attaches to a carbon in the interior of the carbon chain. The following table shows a range of fatty alcohols with their corresponding number of carbon atoms.

**Table 1**

| | | | |
|---|---|---|---|
| Capryl alcohol | 8 | Stearyl alcohol | 18 |
| Capric alcohol | 10 | Arachidyl alcohol | 20 |
| Lauryl alcohol | 12 | Behenyl alcohol | 22 |
| Myristyl alcohol | 14 | Lignoceryl alcohol | 24 |
| Cetyl alcohol | 16 | | |

As the number of carbon atoms decreases, the melting point of the fatty alcohol also decreases. For example, a fatty alcohol with 14 carbon atoms melts at about 100°C, and one with 12 carbon atoms will melt at about 24°C (room temperature). In designing an apertured formed film for a specific use of an absorbent article, it will be apparent to the designer the temperature limit of the application. If the fatty alcohol suspended within the film becomes too low in viscosity, as by melting, it may not remain suspended in the film as is needed. As the number of carbon atoms increases, it may become too viscous for processing in an extrusion process. The preferred range of carbon atoms of fatty alcohol is from 16 to 18.

Many aperture shapes and pattern arrays of apertures are well known in the apertured formed film art. They are designed for the purposes of enhancing fluid transmission as well as imparting aesthetic and tactile properties to the film. For example, US 4324426 teaches how the Equivalent Hydraulic Diameter of the aperture can influence liquid transmission; US 4342314 teaches how to achieve a unique fiber-like aesthetic; and US 4463045 teaches the use of small surface aberrations to impart a cloth-like tactile impression. All of the shapes and pattern arrays of these references and others known in the art can be used in connection with the present embodiments.

For purposes of illustration only, films were made having pentagonal shaped apertures nested side to side such that the apertures are separated by a narrow fiber-like element. The films comprised approximately 90 apertures per square centimeter with an open area of about 20-25 percent and a loft of about 500-650 microns. Loft is the total thickness of the formed film, including the height of the apertures and any surface aberrations. The films had a basis weight of 21.7 grams/m² and a random matte finish. The films were prepared using a direct cast vacuum forming process. The films were made using a polyolefin blend, specifically polyethylene, which is normally hydrophobic. In one film, the blend contained approximately 17,500 ppm to 20,000 ppm of cetyl alcohol. The films described in this paragraph are not included in the scope of the present claims.

The run off value for these films was measured using the procedure stated in US 4456570. The test fluid was the mixture of distilled water, sodium chloride and Triton X-100 (a non-ionic surfactant from Dow Chemical Company) disclosed therein at column 6, lines 10-16. The run off values ranged from 0% to 2.5%. Larger concentrations of cetyl alcohol would insure consistent 0% run off. A concentration of 10,000 ppm yields run off values between 10 and 15 %, and should be considered the lower limit for a diaper topsheet. When designing absorptive devices for other applications with various liquid viscosities and surface tensions, adding a fatty alcohol in amounts as low as about 500 ppm may be sufficient depending on the type of liquid being transmitted and absorbed.

Surprisingly, repeating the testing using stearyl alcohol did not produce similar results. A control film made from a hydrophobic polyolefin (polyethylene) blend with no surfactant additives has a run off value of greater than 80%. Adding stearyl alcohol alone to the blend in amounts up to about 20,000 ppm did not yield run off values less than 78%. However, when added in a 50% to 50% mixture with cetyl alcohol at a combined concentration of 20,000 ppm, a 0% run off value was obtained. Because the cetyl alcohol is the more effective component, blends departing from the 50% to 50% ratio should favor the cetyl alcohol as the majority fatty alcohol in the mixture of cetyl alcohol and stearyl alcohol as defined in claim 1.

The fatty alcohols can be added to the extrusion blend neat or as a compound let down in any polymer known to be missive in the base blend of the intended product. This type of compounding is common in the film extrusion industry. Any compatible thermoplastic, film forming polymer may be used to advantage. Polyolefins, in particular polyethylene and polypropylene, are preferred, especially for films intended for hygiene and personal care applications.

## Claims

1. An extruded hydrophilic apertured formed film for transmitting a liquid
in an absorbent device comprising surface structures that extend from the film surface and terminate in an aperture, the film further comprising a blend of at least one thermoplastic olefin polymer and fatty alcohol, **characterized in that** the fatty alcohol is a mixture of cetyl alcohol and stearyl alcohol.

2. The film of claim 1 wherein the fatty alcohol is present in the amount of at least about 500 ppm.

3. The film of claim 3 wherein the fatty alcohol is present in the amount of 10,000-20,000 ppm.

4. The film of claim 5 wherein mixture comprises at least 50% cetyl alcohol.

5. The film of claim 5 wherein the fatty alcohol is present in the amount of at least about 20,000 ppm.

6. An absorbent article comprising a topsheet and an absorbent core, said topsheet comprising the film of any of claims 1-5.

7. An absorbent article comprising a topsheet, an absorbent core and a transfer layer positioned between the topsheet and the core, wherein at least one of the topsheet and the transfer layer comprises a film according to any one of claims 1-5.

## Patentansprüche

1. Extrudierte hydrophile, mit Öffnungen ausgebildete, Folie, um eine Flüssigkeit in eine saugfähige Einrichtung zu befördern, umfassend Oberflächenstrukturen, die sich von der Folienoberfläche aus erstrecken und in einer Öffnung enden, wobei die Folie des Weiteren eine Mischung aus wenigstens einem thermoplastischen Olefinpolymer und Fettalkohol umfasst, **dadurch gekennzeichnet, dass** der Fettalkohol eine Mischung aus Cetylalkohol und Stearyolalkohol ist.

2. Folie nach Anspruch 1, wobei der Fettalkohol in der Menge von wenigstens ungefähr 500 ppm vorhanden ist.

3. Folie nach Anspruch 3, wobei der Fettalkohol in der Menge von 10.000 bis 20.000 ppm vorhanden ist.

4. Folie nach Anspruch 5, wobei die Mischung wenigstens 50% Cetylalkohol umfasst.

5. Folie nach Anspruch 5, wobei der Fettalkohol in der Menge von wenigstens ungefähr 20.000 ppm vorhanden ist.

6. Absorptionsgegenstand umfassend eine Deckschicht und einen saugfähigen Kern, wobei die Deckschicht die Folie nach einem der Ansprüche 1 bis 5 enthält.

7. Absorbierender Gegenstand umfassend eine Deckschicht, einen saugfähigen Kern und eine Transferschicht, angeordnet zwischen der Deckschicht und dem Kern, wobei wenigstens eine der Deckschicht und der Transferschicht eine Folie gemäß einem der Ansprüche 1 bis 5 umfasst.

## Revendications

1. Film façonné ajouré hydrophile extrudé destiné à transmettre un liquide dans un dispositif absorbant comprenant des structures de surface qui s'étendent à partir de la surface du film et se terminent dans une ouverture, le film comprenant en outre un mélange d'au moins un polymère d'oléfine thermoplastique et d'un alcool gras, **caractérisé en ce que** l'alcool gras est un mélange d'alcool cétylique et d'alcool stéarylique.

2. Film selon la revendication 1, dans lequel l'alcool gras est présent dans la quantité d'au moins environ 500 ppm.

3. Film selon la revendication 3, dans lequel l'alcool gras est présent dans la quantité de 10 000 à 20 000 ppm.

4. Film selon la revendication 5, dans lequel le mélange comprend au moins 50 % d'alcool cétylique.

5. Film selon la revendication 5, dans lequel l'alcool gras est présent dans la quantité d'au moins environ 20 000 ppm.

6. Article absorbant comprenant une couche supérieure et un coeur absorbant, ladite couche supérieure comprenant le film selon l'une quelconque des revendications 1 à 5.

7. Article absorbant comprenant une couche supérieure, un coeur absorbant et une couche de transfert positionnée entre la couche supérieure et le coeur, dans lequel au moins l'un de la couche supérieure et de la couche de transfert comprend un film selon l'une quelconque des revendications 1 à 5.
